# EUROPEAN PATENT SPECIFICATION

(11) **EP 3 675 909 B1**
(45) Date of publication and mention of the grant of the patent: **18.10.2023**
(21) Application number: 18768797.5
(22) Date of filing: 31.08.2018
(51) Int. Cl.: A61K 45/00, A61P 19/00

(54) **IMMUNMODULATION FOR PREVENTION OF POOR HEALING OF MUSCULOSKELETAL INJURIES IN COMPROMISED PATIENTS**
IMMUNMODULATION ZUR VERHINDERUNG DER SCHLECHTEN HEILUNG VON MUSKEL-SKELETT-LÄSIONEN BEI BEEINTRÄCHTIGTEN PATIENTEN
IMMUNOMODULATION POUR LA PRÉVENTION D'UNE MAUVAISE CICATRISATION DE LÉSIONS MUSCULO-SQUELETTIQUES CHEZ DES PATIENTS FRAGILISÉS

(30) Priority: 31.08.2017 EP 17188813
(43) Date of publication of application: 08.07.2020
(73) Proprietor: Charité Universitätsmedizin Berlin, 10117 Berlin (DE)
(72) Inventor: SCHMIDT-BLEEK, Katharina, 12165 Berlin (DE); VOLK, Hans-Dieter, 10117 Berlin (DE); WENDLER, Sebastian, 10249 Berlin (DE); DUDA, Georg N., 12209 Berlin (DE); REINKE, Simon, 10823 Berlin (DE); GEISSLER, Sven, 10551 Berlin (DE); QAZI, Talmoor Hasan, Philadelphia 19146, PA (US); DIENELT, Anke, 13469 Berlin (DE); SCHELL, Hanna, 10551 Berlin (DE)
(74) Representative: Schulz Junghans Patentanwälte PartGmbB
(86) International application number: PCT/EP2018/073453
(87) International publication number: WO 2019/043148

(56) References cited:
- US-A1- 2015 164 853
- DOGAN ALI ET AL: "Iloprost inhibits fracture repair in rats", CHINESE MEDICAL JOURNAL, BEIJING: CHINESE MEDICAL ASSOCIATION; MUMBAI: WOLTERS KLUWER HEALTH, CN, vol. 127, no. 16, 20 August 2014 (2014-08-20), pages 2960-2965, XP009543466, ISSN: 2542-5641, DOI: 10.3760/CMA.J.ISSN.0366-6999.20132862

## Description

The present invention relates to a compound for use in a method for healing a musculoskeletal injury or preventing a delayed healing of a musculoskeletal injury, particularly in compromised patients.

### Background of the invention

Musculoskeletal injuries encompass medical conditions of the locomotor system such as bone fractures, torn or otherwise damaged muscles or ruptured tendons. Musculoskeletal injuries often result from accidents or strenuous activity. Studies have approximated that up to 25% of the population may experience some sort of musculoskeletal injury within a year. For example, in Germany the number of bone fracture incidents is estimated to be 1.6 million per year. Long healing times for these injuries decrease the quality of life of the patient, increase the cost of medical care and lead to longer medical leave.

Frequently, delayed healing or non-healing situations require second interventions, which substantially impact quality of life and further increase health care costs. In young patients, revisions delay the return to work; in aged patients, revisions often impair functional recovery and can therefore represent a threat to the general health status of aged patients, sometimes even with lethal consequences.

As musculoskeletal injuries therefore represent a considerable burden on society and the economy, it is crucial to identify the patients at risk in advance and use immunomodulatory therapies that substantially improve the healing process.

Based on this background it is the objective of the present invention to provide means for improved treatment of musculoskeletal injuries or improved prevention of delayed healing or non-healing of musculoskeletal injuries, particularly in compromised patients.

### Description of the invention

This objective is attained by a compound having the features of claim 1, a composition having the features of claim 10 and an implant having the features of claim 14. Preferred embodiments are described in the sub claim and in detail below.

According to claim 1, a compound for use in a method for treating a musculoskeletal injury or preventing a delayed healing or non-healing of a musculoskeletal injury is provided, wherein the compound is a regulator of the pro-inflammatory response.

Particularly, the compound of the invention acts as a immune modulator capable of upregulating regulatory T cells and/or M2-macrophages and/or as immune modulator capable of downregulating the biological activity of effector CD8+ cells.

Preferably, both effects are mediated by the same compound.

Particularly, the compound of the invention is administered according to the following dosage regimen:
- administering an initial dose of the compound of the invention to a patient not before 24 hours after the musculoskeletal injury.

The skilled person will understand that the initial dose is administered not before 24 hours after the musculoskeletal injury occurred.

In certain embodiments, the initial dose of the compound of the invention is administered not before 36 hours after the musculoskeletal injury.

The term "upregulating regulatory T cells and/or M2-macrophages" in the context of the present specification particularly refers to upregulating the biological activity of regulatory T cells and/or M2-macrophages, particularly the ability of the regulatory T cells and/or M2-macrophages to decrease or inhibit the pro-inflammatory response or the initial inflammation reaction after the musculoskeletal injury.

The term "downregulating the biological activity of effector CD8+ cells" particularly refers to decrease or inhibit the ability of effector CD8+ cells of initiating or maintaining the pro-inflammatory response or the initial inflammation reaction after musculoskeletal injury.

The term "immune modulator" in the context of the present specification particularly refers to a compound or substance that enhances or decreases the biological activity or function of the respective target cells.

The term "regulatory T cells" is used in its meaning known in the art of immunology. It particularly refers to CD4+CD25+FOXP3+ cells. Likewise, the term "effector CD8+ cells" is used in its meaning known in the art of immunology. Non-limiting examples for effector C8+ cells include effector memory CD8+ cells, particularly CD8 TEMRA cells (CD8+1 1 a++28-57+).

In certain embodiments, the compound of the invention is an anti-inflammatory agent.

The term "anti-inflammatory agent in the context of the present specification particularly refers to compound or a substance that reduces inflammation or swelling.

In certain embodiments, the compound of the invention is selected from:
- a prostacyclin
- a phosphodiesterase inhibitor IV,
- dibutryl-cAMP, or
- a CD39/CD73 agonist, particularly capable of supporting the endogenous generation of adenosine/cAMP.

In certain embodiments, the prostacyclin is Iloprost (CAS No 78919-13-8).

In certain embodiments, the phosphodiesterase inhibitor IV is selected from:
- Rolipram (CAS No 61413-54-5);
- Apremilast (CAS No 608141-41-9);
- Cilomilast (CAS No 153259-65-5);
- Crisaborole (CAS 906673-24-3);
- Diazepam (CAS No 439-14-5);
- Ibudilast (CAS No 50847-11-5);
- Luteolin (CAS N0491-70-3);
- Mesembrenone (CAS No 468-54-2); or
- Piclamilast (CAS No 144035-83-6).

In certain embodiments, the initial dose is administered to the patient between 3 d to 4 d after the musculoskeletal injury.

In certain embodiments, the compound of the invention is administered to the patient not longer than 7 d after the musculoskeletal injury. In certain embodiments, the compound of the invention is administered to the patient not longer than 6 d after the musculoskeletal injury.

In certain embodiments, the compound of the invention is administered within a period of 2 d to 7 d after the musculoskeletal injury. In certain embodiments, the compound of the invention is administered within a period of 2 d to 6 d after the musculoskeletal injury.

In certain embodiments, the patient is compromised. The term "compromised patient" in the context of the present specification particularly is defined as a patient with enhanced age, osteoporosis, additional co-morbidities (e.g. metabolic disorders such as diabetes, cachexia) but also immune disbalances (enhanced effector T cell/regulatory t cell ratio), typically seen in "immunoaged" patients. A compromised patient particularly has a reduced regenerative capacity illustrated by a higher incidence of delayed or incomplete posttraumatic musculoskeletal healing.

In certain embodiments, the musculoskeletal injury is selected from bone fracture, tendon injury, tendon rupture, or torn or damaged muscle.

According to claim 10, a pharmaceutical composition for use in a method for treating a musculoskeletal injury or for preventing a delayed healing of a musculoskeletal injury is provided, wherein the composition comprises a compound according to the above aspect or any one of the above embodiments.

In certain embodiments, the pharmaceutical composition of the invention is formulated for injection or implantation.

In certain embodiments, the pharmaceutical composition of the invention further comprises a biodegradable polymer or a mixture of biodegradable polymers.

The term "biodegradable polymer" in the context of the present specification particularly refers to a polymer that is degraded or decomposed in the target environment, for example in the vicinity of a broken bone or a torn or damaged muscle. Non-limiting examples of suitable biodegradable polymers include fibrin and collagen.

In certain embodiments, the pharmaceutical composition of the invention is embedded in the biodegradable polymer or the mixture of biodegradable polymers. In certain embodiments, the pharmaceutical composition embedded in the biodegradable polymer or the mixture of biodegradable polymers is coated with the same biodegradable polymer or mixture or with a different biodegradable polymer or mixture of biodegradable polymers. In certain embodiments, the pharmaceutical composition embedded in the biodegradable polymer or the mixture of biodegradable polymers is surrounded by a shell comprising or consisting of the same biodegradable polymer or mixture or with a different biodegradable polymer or mixture.

According to claim 14, an implant, particularly an orthopaedic implant is provided, wherein the implant comprises the composition of the invention, which is particularly formulated for implantation according to the above embodiments.

Accordingly, the composition is preferably embedded in the biodegradable polymer or the mixture of biodegradable polymers as described above. More preferable, the composition embedded in the biodegradable polymer of the mixture of biodegradable polymers is coated with the same biodegradable polymer or mixture or with a different biodegradable polymer or mixture or surrounded by a shell comprising or consisting of the same biodegradable polymer or mixture or with a different biodegradable polymer or mixture .

Also within the scope of the invention is a method for treating a musculoskeletal injury or for preventing a delayed healing of a musculoskeletal injury. The method comprises administering a compound according to the first aspect of the invention or any one of the embodiments thereof or a composition according to the above aspect or any one of the embodiments thereof in a pharmaceutically effective concentration to a patient in need thereof not before 24 hours after said musculoskeletal injury.

Particularly, the method for treating a musculoskeletal injury or for preventing a delayed healing of a musculoskeletal injury comprises the administration of the compound of the invention according to the above described dosage regimen.

Wherever alternatives for single separable features are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.

### Description of the figures

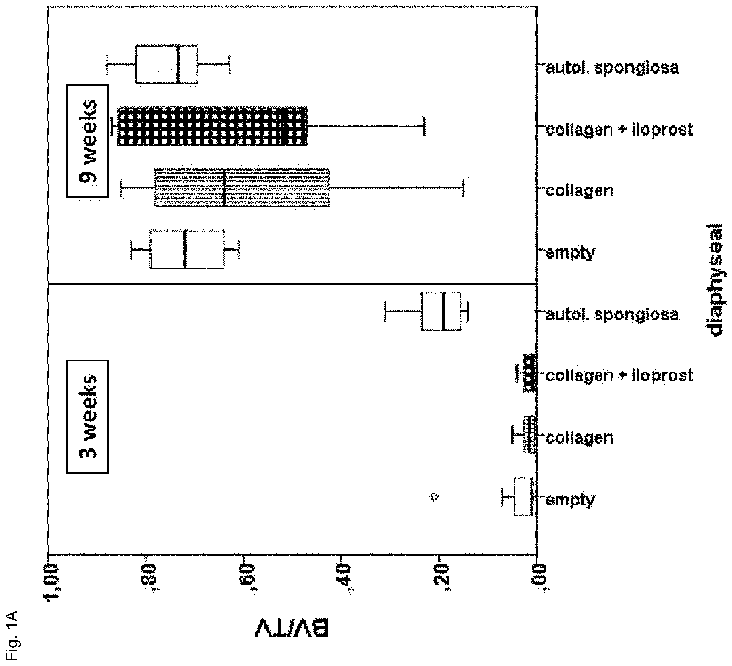

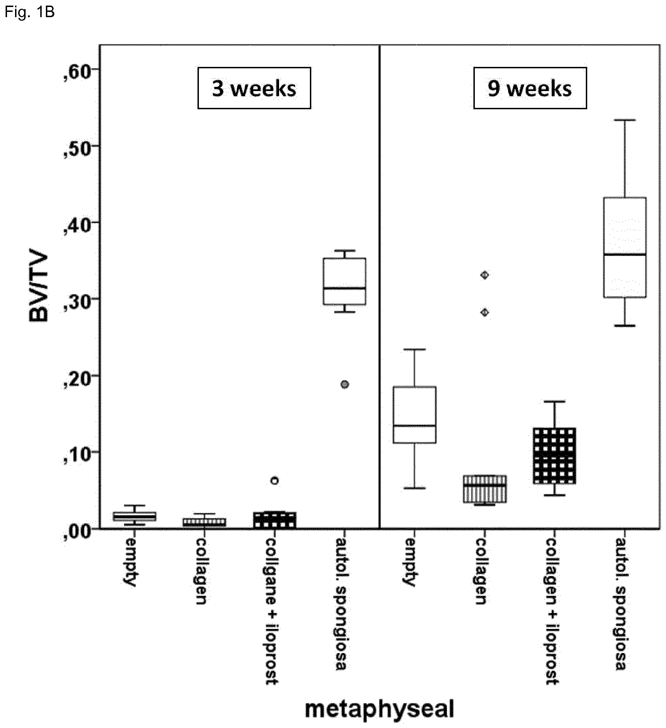

- Fig. 1: shows a Micro Computer Tomography (µCT) evaluation of bone formation in a drill hole in the diphyseal (A) and metaphyseal (B) bone (sheep) after 3 and 9 weeks. No differences between the collagen group and the collagen Iloprost loaded group can be detected. In the collagen Iloprost loaded group healing has i) not progressed as far as in the group that received autologous spongiosa and is ii) also slightly worse than the control due to the carrier (seen in the gelatin only group) that has to be degraded before new bone can be formed.

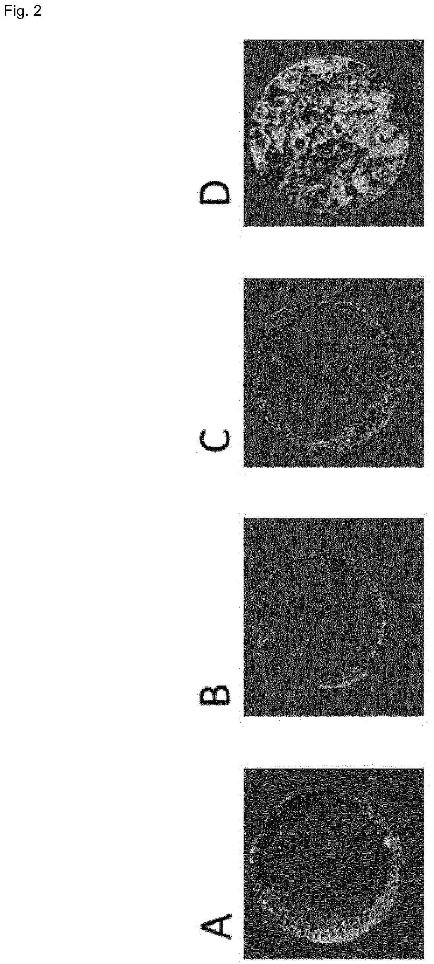

- Fig. 2: shows a 3D reconstruction: A control, B collagen only, C collagen loaded with Iloprost, and D autologous spongiosa after 3 weeks of healing.

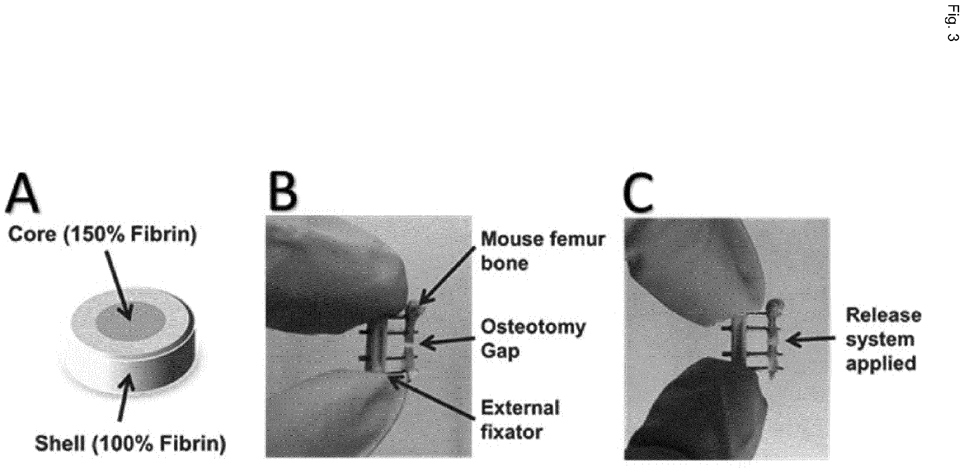

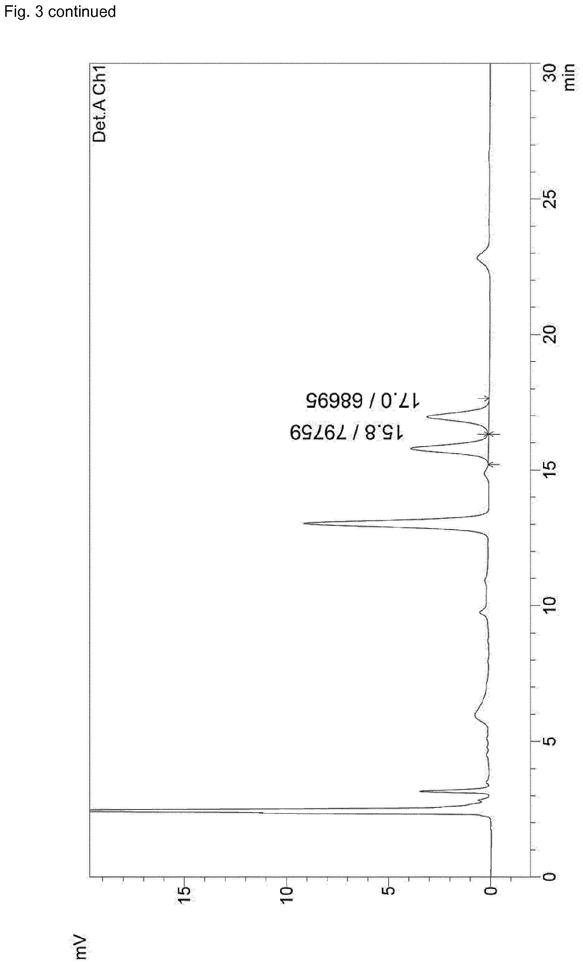

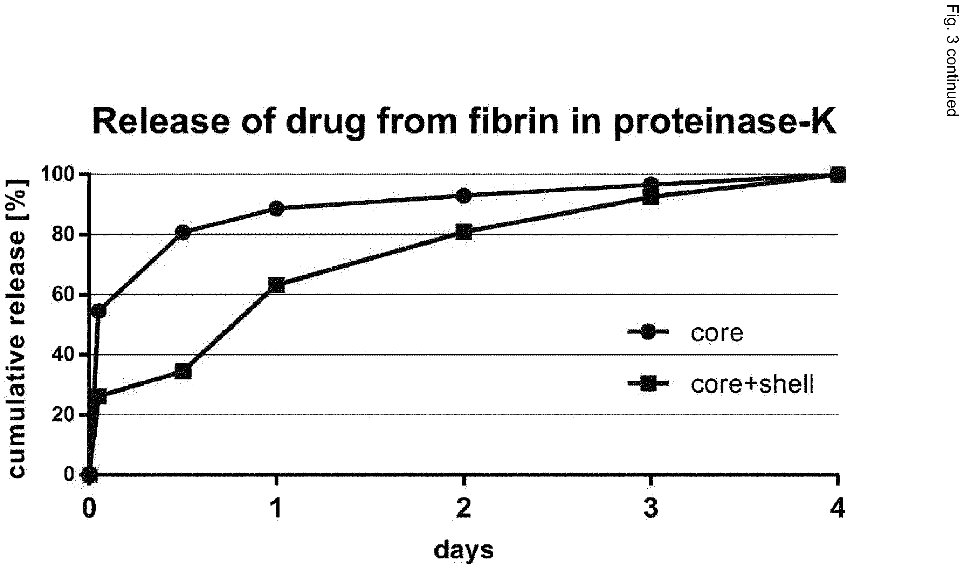

- Fig. 3: shows the structure and function of a preferred the drug release system: (A): Prior to application the drug was incorporated into the core of the Fibrin-based release system. A protective shell of 100% Fibrin of the applied kit (Tissuecol, Baxter) was constructed around the drug-loaded core of 150% Fibrin. (B): empty mouse osteotomy specimen and (C): with applied release system. (D): HPLC/UV chromatogram of the drug with its characteristic double peaks. the drug was separated from protein residues via liquid-liquid extraction and analyzed with a C-18 column. (E): The drug release was done in PBS supplemented with Proteinase-K. Double-peak quantification showed a delayed release of the drug from the hydrogel with the surrounding shell.

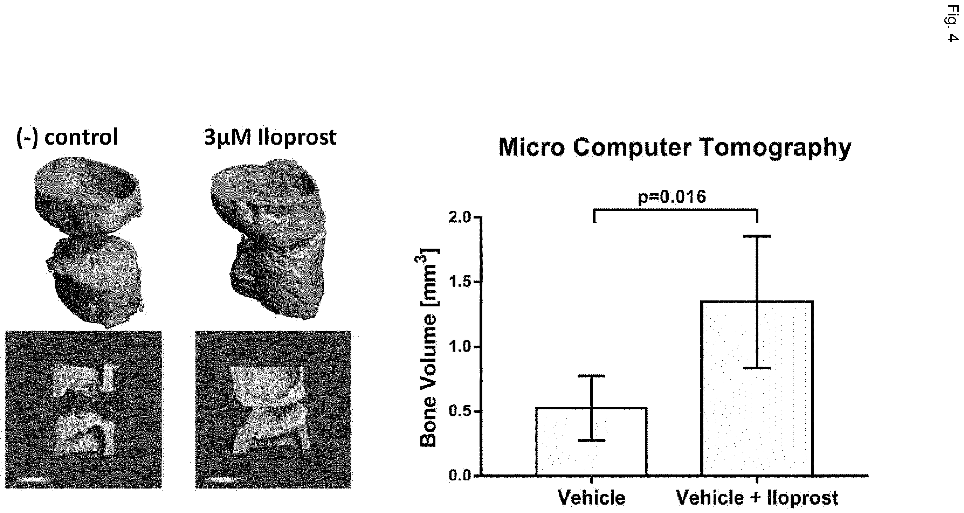

- Fig. 4: shows the immunomodulation *in vivo* via Micro Computer Tomography (µCT) of femur mouse osteotomies after a healing period of 21 days. The drug loaded release system achieved a higher amount of newly formed bone volume (BV), i.e. vehicle 0.436 mm³ and vehicle+lloprost 1.347 mm³ . N = 6 each sample type, median, Mann-Whitney-U test (p = 0.016).

### Examples:

Particularly disclosed in here is a composition for use in a method for prophylactic treatment of compromised patient suffering on a musculoskeletal injury.

This prophylactic treatment is an immune modulation, for example, by upregulation of Treg and/or M2 macrophages and/or downregulation of CD8 via an anti-inflammatory agent (like a phosphodiesterase IV inhibitor, dibutyryl cAMP, Iloprost (a prostacyclin compound) or a CD39/CD73 agonist, or a combination thereof.

Surprisingly, the inventors found that the treatment must take place during a limited time window after the initial inflammation reaction, which is crucial in the triggering of bone repair mechanisms. The anti-inflammatory agent then reduces the inflammation or promotes the immune system to switch from an inflammatory to an anti-inflammatory response at the site of injury.

The method may include the selection of an appropriate patient group via TEMRA-CD8 or CD4/CD8 ratio (for example according to a method as disclosed in PCT/EP2013/052181) and any person above 65 years of age.

Surprisingly, the inventors found - in experiments in sheep - that the treatment must not take place early after the injury (usually within 24 h). Instead, they determined that only treatment at least 24-36 h after the injury is effective. Furthermore, the inventors found that treatment doesn't have to be continued for more than 7 days, because longer treatment does not improve healing outcomes. This treatment regime is reducing the exposure time of the patient to the treatment and therefore reduces possible side effects.

Preferably, the treatment should take place only between days 3 and 4 after injury to terminate the pro-inflammatory phase and to support the subsequent anti-inflammatory phase.

This treatment can be given by injection or implantation or via a release system.

The inventors used a local slow-release system that releases the anti-inflammatory agent almost completely between days 2 and 6 after injury. The release system was loaded with an anti-inflammatory agent and released less than 35% of the agent within 24 hours, but released 80% of the agent within 60 hours. Preferably, the release is completed (>90%) within 7 days after injury.

The inventors could show that healing of musculoskeletal injuries with this method is much better than when applied too early. It is crucial to apply the treatment in the appropriate phase of bone healing. As such, this method could be especially helpful for improving the healing of musculoskeletal injuries in compromised patients.

The release system can be used for all musculoskeletal injuries like bone fractures, tissue injuries, wounds, etc.

### Sheep experiments

Prior publications, e.g. US 20150164853 A1, stated that immediate use of Iloprost locally to enhance bone healing is beneficial. To investigate this a high dose of Iloprost was used in a sheep bone healing model where the agent was applied in a collagen scaffold, which has a burst-release dynamic. However, no enhanced bone formation was detectable in the performed study. Without wishing to be bound by theory, the inventors believe that the primary pro-inflammatory burst is swiftly downregulated to give way to a more anti-inflammatory-pro-regenerative signalling - this result strongly indicates that a time delayed application of Iloprost is essential for a positive effect on the bone healing process.

Bone formation has been analyzed in a drill hole model (Establishment of a preclinical ovine screening model for the investigation of bone tissue engineering strategies in cancellous and cortical bone defects. Pobloth AM, Johnson KA, Schell H, Kolarczik N, Wulsten D, Duda GN, Schmidt-Bleek K. BMC Musculoskelet Disord. 2016 Mar 1;17:111.). The empty control group and the collagen only group were used as negative controls while the autologous spongiosa group, the current gold standard in bone therapy, was used as a positive control. In the Ilomedin group a supraphysiological dosis of 1 ml Iloprost (Ilomedin, 20 µl/1 ml Infusionslösung, Bayer Vital GmbH, Leverkusen, Deutschland) per two scaffolds was used.

### Mouse experiments

Mouse experiments were performed with 12-week-old C57BL/6 females (n = 6, each sample type) (Charles River Laboratories) according to the policies and principles established by the Animal Welfare Act, the National Institutes of Health Guide for the Care and Use of Laboratory Animals, and the National Animal Welfare Guidelines. All animal experiments were approved by the local legal representative. Animals were kept under obligatory hygiene standards that were monitored according to the FELASA standards.

The osteotomy was performed on the left femur. After shaving and disinfecting the operation area of isoflurane-anesthetized animals, a lateral longitudinal incision of the skin (2mm) from knee to hip was performed for a mid-diaphyseal approach to the femur. The femur was exposed by blunt preparation of *Musculus vastus lateralis* and *Muscculus biceps femoris,* carefully sparing the sciatic nerve. Serial drilling for pin placement (0.45 mm diameter) through the connector bar of the external fixator (MouseExFix, RISystem, Davos, Switzerland) was performed, thus positioning the external fixator laterally in parallel to the femur. A 0.70mm osteotomy was performed between the middle pins using a Gigli wire saw (RISystem, Davos, Switzerland). Subsequently, the release system was applied, see below. After skin closure, mice were returned to their cages, postoperative analgesia was conducted with tramadol hydrochloride added to the drinking water (25mg/L). Animals were euthanized after 21 days healing period.

### Release system

Fibrinogen and Thrombin-S solutions (both Tissucol-kit Immuno, Baxter) where heated to 37°C. 3 µl Fibrinogen (16 mg/100 µl) were added to 1 µl Thrombin-S (after manufactures protocol) and polymerized for 2 min to prepare the shell gel. In parallel, the core gel was generated with 3 µl Fibrinogen (24 mg/100 µl) which were added to 1 µl Thrombin-S and polymerized for 2 min. This core gel contained either 3 µM Iloprost (20µg/ml Ilomedin, Bayer) or 5,4µl PBS (Gibco) for treatment and control, respectively. Afterwards, this core gel was placed into the shell gel and subsequently completed with another addition of 3 µl Fibrinogen (16 mg/100 µl), which were added to 1 µl Thrombin-S. After 2 min of polymerization, the release system was carefully placed into the osteotomy gap with a sterile tweezer. Finally, the hydrogel composition was glued to the bone tissue using 6 µl Fibrinogen (16 mg/100 µl) and 2 µl Thrombin-S. After 2 min of polymerization the wound was stitched.

### µCT analysis

The newly formed mineralized bone tissues were analyzed using high-resolution micro computed tomography (µCT). After fixation via 4% PFA/PBS and dehydration with a sucrose gradient, the fractures were scanned with a fixed isotropic voxel size of 10.5-m (Viva40 micro-Cl, Seaneo Medical AG", Switzerland, 70KVp, 114µA) . The scan axis coincided with the diaphyseal axis of the femora. A minimum of 190 slices a 10.5~m was chosen such that the fracture callus was completely included. The cortical bone was manually excluded from the volume of interest (VOI) in further post-processing. A fixed global threshold of 240 mg HA/cm³ was selected that allowed the rendering of mineralized tissue only. All analyses were performed on the digitally extracted callus tissue using 3D distance techniques (Scanco software, Switzerland).

### Statistical analysis

Unless otherwise stated, all data were represented as median ± SD. The Mann-Whitney U test was used for comparison between the groups. The statistical analyses were performed with SPSS 18.

### Results: Mouse experiments

*In vivo* results showed significantly increased volumes of newly formed bone after 21 days of regeneration of Iloprost loaded release systems in comparison to the empty vehicle control. These data were derived from micro computer tomography (µCT) scans.

## Claims

1. A compound for use in a method for treating a musculoskeletal injury or preventing a delayed healing or non-healing of a musculoskeletal injury, wherein said compound is a regulator of the pro-inflammatory response, and wherein particularly said compound is capable of
- upregulating regulatory T cells and/or M2-macrophages; and/or
- downregulating the biological activity of effector CD8+ cells and further comprising the dosage regimen:
- administering an initial dose of said compound to a patient not before 24 hours after said musculoskeletal injury.

2. The compound for use in a method for treating a musculoskeletal injury or preventing a delayed healing or non-healing of a musculoskeletal injury according to claim 1, wherein said compound is selected from
- a prostacyclin;
- a phosphodiesterase IV inhibitor;
- dibutyryl-cAMP; or
- a CD39/CD73 agonist.

3. The compound for use in a method treating a musculoskeletal injury or preventing a delayed healing or non-healing of a musculoskeletal injury according to claim 1 or 2, wherein said compound is Iloprost.

4. The compound for use in a method for treating a musculoskeletal injury or preventing a delayed healing or non-healing of a musculoskeletal injury according to claim 1 or 2, wherein said compound is selected from Rolipram, Apremilast, Cilomilast, Crisaborole, Diazepam, Ibudilast, Luteolin, Mesembrenone or Piclamilast.

5. The compound for use in a method for treating a musculoskeletal injury or preventing a delayed healing or non-healing of a musculoskeletal injury according to any one of the preceding claims, wherein said initial dose is administered to said patient not before 36 hours after said musculoskeletal injury.

6. The compound for use in a method for treating a musculoskeletal injury or preventing a delayed healing or non-healing of a musculoskeletal injury according to any one of the preceding claims, wherein said initial dose is administered to said patient between 3 d to 4 d after said musculoskeletal injury.

7. The compound for use in a method for treating a musculoskeletal injury or preventing a delayed healing or non-healing of a musculoskeletal injury according to any one of the preceding claims, wherein said compound is administered to said patient not longer than 7 d after said musculoskeletal injury, particularly not longer than 6 d after said musculoskeletal injury.

8. The compound for use in a method for treating a musculoskeletal injury or preventing a delayed healing or non-healing of a musculoskeletal injury according to any one of the preceding claims, wherein said patient is compromised.

9. The composition for use in a method for treating a musculoskeletal injury or preventing a delayed healing or non-healing of a musculoskeletal injury according to any one of the preceding claims, wherein said musculoskeletal injury is selected from bone fracture, tendon injury, tendon rupture, torn or damaged muscle.

10. A pharmaceutical composition for use in a method for treating a musculoskeletal injury or preventing a delayed healing or non-healing of a musculoskeletal injury comprising a compound according to any one of the preceding claims.

11. The composition for use in a method for treating a musculoskeletal injury or preventing a delayed healing or non-healing of a musculoskeletal injury according claim 10, wherein said composition is formulated for injection or implantation.

12. The composition for use in a method for treating a musculoskeletal injury or preventing a delayed healing or non-healing of a musculoskeletal injury according to claim 10 or 11, wherein said composition further comprises a biodegradable polymer, particularly fibrin.

13. The composition for use in a method for treating a musculoskeletal injury or preventing a delayed healing or non-healing of a musculoskeletal injury according to claim 12, wherein said composition is embedded in said biodegradable polymer and optionally coated with said biodegradable polymer or another biodegradable polymer, or substantially surrounded by a shell comprising or consisting of said biodegradable polymer or another biodegradable polymer.

14. An implant, particularly an orthopaedic implant, comprising a composition according to any one of claims 10 to 13.

## Patentansprüche

1. Eine Verbindung zur Verwendung in einem Verfahren zur Behandlung einer Muskel-Skelett-Verletzung oder Verhinderung einer verzögerten Heilung oder Nichtheilung einer Muskel-Skelett-Verletzung, wobei besagte Verbindung ein Regulator der proentzündlichen Reaktion ist, und wobei insbesondere besagte Verbindung dazu in der Lage ist
- regulatorische T-Zellen und/oder M2-Makrophagen hoch zu regulieren; und/oder
- biologische Aktivität von CD8+-Effektorzellen herunter zu regulieren und weiterhin umfassend das Dosierungsregime:
- Verabreichen einer Initialdosis besagter Verbindung an einen Patienten frühestens 24 Stunden nach der Muskel-Skelett-Verletzung.

2. Die Verbindung zur Verwendung in einem Verfahren zur Behandlung einer Muskel-Skelett-Verletzung oder Verhinderung einer verzögerten Heilung oder Nichtheilung einer Muskel-Skelett-Verletzung gemäß Anspruch 1, wobei die Verbindung ausgewählt ist aus
- einem Prostazyklin;
- einem Phosphodiesterase-IV-Inhibitor;
- Dibutyryl-cAMP; oder
- einem CD39/CD73-Agonisten.

3. Die Verbindung zur Verwendung in einem Verfahren zur Behandlung einer Muskel-Skelett-Verletzung oder Verhinderung einer verzögerten Heilung oder Nichtheilung einer Muskel-Skelett-Verletzung gemäß Anspruch 1 oder 2, wobei die Verbindung Iloprost ist.

4. Die Verbindung zur Verwendung in einem Verfahren zur Behandlung einer Muskel-Skelett-Verletzung oder Verhinderung einer verzögerten Heilung oder Nichtheilung einer Muskel-Skelett-Verletzung gemäß Anspruch 1 oder 2, wobei die Verbindung ausgewählt ist aus Rolipram, Apremilast, Cilomilast, Crisaborol, Diazepam, Ibudilast, Luteolin, Mesembrenon oder Piclamilast.

5. Die Verbindung zur Verwendung in einem Verfahren zur Behandlung einer Muskel-Skelett-Verletzung oder Verhinderung einer verzögerten Heilung oder Nichtheilung einer Muskel-Skelett-Verletzung gemäß einem der vorhergehenden Ansprüche, wobei besagte Initialdosis dem Patienten frühestens 36 Stunden nach besagter Muskel-Skelett-Verletzung verabreicht wird.

6. Die Verbindung zur Verwendung in einem Verfahren zur Behandlung einer Muskel-Skelett-Verletzung oder Verhinderung einer verzögerten Heilung oder Nichtheilung einer Muskel-Skelett-Verletzung gemäß einem der vorhergehenden Ansprüche, wobei besagte Initialdosis besagtem Patienten zwischen 3 d bis 4 d nach besagter Muskel-Skelett-Verletzung verabreicht wird.

7. Die Verbindung zur Verwendung in einem Verfahren zur Behandlung einer Muskel-Skelett-Verletzung oder Verhinderung einer verzögerten Heilung oder Nichtheilung einer Muskel-Skelett-Verletzung gemäß einem der vorhergehenden Ansprüche, wobei besagte Verbindung dem Patienten nicht länger als 7 Tage nach besagter Muskel-Skelett-Verletzung, insbesondere nicht länger als 6 Tage nach besagter Muskel-Skelett-Verletzung verabreicht wird.

8. Die Verbindung zur Verwendung in einem Verfahren zur Behandlung einer Muskel-Skelett-Verletzung oder Verhinderung einer verzögerten Heilung oder Nichtheilung einer Muskel-Skelett-Verletzung gemäß einem der vorhergehenden Ansprüche, wobei besagter Patient beeinträchtigt ist.

9. Die Zusammensetzung zur Verwendung in einem Verfahren zur Behandlung einer Muskel-Skelett-Verletzung oder Verhinderung einer verzögerten Heilung oder Nichtheilung einer Muskel-Skelett-Verletzung gemäß einem der vorhergehenden Ansprüche, wobei besagte Muskel-Skelett-Verletzung ausgewählt ist aus Knochenbruch, Sehnenverletzung, Sehnenriss, gerissenem oder beschädigtem Muskel.

10. Eine pharmazeutische Zusammensetzung zur Verwendung in einem Verfahren zur Behandlung einer Muskel-Skelett-Verletzung oder Verhinderung einer verzögerten Heilung oder Nichtheilung einer Muskel-Skelett-Verletzung, umfassend eine Verbindung gemäß einem der vorangehenden Ansprüche.

11. Eine Zusammensetzung zur Verwendung in einem Verfahren zur Behandlung einer Muskel-Skelett-Verletzung oder Verhinderung einer verzögerten Heilung oder Nichtheilung einer Muskel-Skelett-Verletzung gemäß Anspruch 10, wobei besagte Zusammensetzung zur Injektion oder Implantation formuliert ist.

12. Die Zusammensetzung zur Verwendung in einem Verfahren zur Behandlung einer Muskel-Skelett-Verletzung oder Verhinderung einer verzögerten Heilung oder Nichtheilung einer Muskel-Skelett-Verletzung gemäß Anspruch 10 oder 11, wobei die Zusammensetzung weiterhin ein biologisch abbaubares Polymer, insbesondere Fibrin, umfasst.

13. Die Zusammensetzung zur Verwendung in einem Verfahren zur Behandlung einer Muskel-Skelett-Verletzung oder Verhinderung einer verzögerten Heilung oder Nichtheilung einer Muskel-Skelett-Verletzung gemäß Anspruch 12, wobei besagte Zusammensetzung in besagtes biologisch abbaubares Polymer eingebettet und wahlweise mit besagtem biologisch abbaubarem Polymer oder einem anderen biologisch abbaubaren Polymer beschichtet ist oder im Wesentlichen von einer Hülle umgeben ist, die besagtes biologisch abbaubares Polymer oder ein anderes biologisch abbaubares Polymer umfasst oder daraus besteht.

14. Ein Implantat, insbesondere ein orthopädisches Implantat, umfassend eine Zusammensetzung gemäß einem der Ansprüche 10 bis 13.

## Revendications

1. Composé destiné à être utilisé dans un procédé de traitement d'une lésion musculosquelettique ou de prévention d'une cicatrisation retardée ou absence de cicatrisation d'une lésion musculosquelettique, dans lequel ledit composé est un régulateur de la réponse pro-inflammatoire, et dans lequel ledit composé est notamment capable de
- réguler à la hauteur des lymphocytes T régulateurs et/ou macrophages M2 ; et/ou
- réguler à la baisse l'activité biologique de lymphocytes CD8+ effecteurs et comprenant en outre le régime posologique :
- administrer une dose initiale dudit composé à un patient pas avant 24 heures après ladite lésion musculosquelettique.

2. Composé destiné à être utilisé dans un procédé de traitement d'une lésion musculosquelettique ou de prévention d'une cicatrisation retardée ou absence de cicatrisation d'une lésion musculosquelettique selon la revendication 1, dans lequel ledit composé est sélectionné parmi
- une prostacycline ;
- un inhibiteur de phosphodiestérase IV ;
- dibutyryl-cAMP ; ou
- un agoniste CD39/CD73.

3. Composé destiné à être utilisé dans un procédé de traitement d'une lésion musculosquelettique ou de prévention d'une cicatrisation retardée ou absence de cicatrisation d'une lésion musculosquelettique selon la revendication 1 ou 2, dans lequel ledit composé est l'Iloprost.

4. Composé destiné à être utilisé dans un procédé de traitement d'une lésion musculosquelettique ou de prévention d'une cicatrisation retardée ou absence de cicatrisation d'une lésion musculosquelettique selon la revendication 1 ou 2, dans lequel ledit composé est sélectionné parmi Rolipram, Aprémilast, Cilomilast, Crisaborole, Diazépam, Ibudilast, Lutéoline, Mésembrénone ou Piclamilast.

5. Composé destiné à être utilisé dans un procédé de traitement d'une lésion musculosquelettique ou de prévention d'une cicatrisation retardée ou absence de cicatrisation d'une lésion musculosquelettique selon l'une quelconque des revendications précédentes, dans lequel ladite dose initiale est administrée audit patient pas avant 36 heures après ladite lésion musculosquelettique.

6. Composé destiné à être utilisé dans un procédé de traitement d'une lésion musculosquelettique ou de prévention d'une cicatrisation retardée ou absence de cicatrisation d'une lésion musculosquelettique selon l'une quelconque des revendications précédentes, dans lequel ladite dose initiale est administrée audit patient entre 3 j et 4 j après ladite lésion musculosquelettique.

7. Composé destiné à être utilisé dans un procédé de traitement d'une lésion musculosquelettique ou de prévention d'une cicatrisation retardée ou absence de cicatrisation d'une lésion musculosquelettique selon l'une quelconque des revendications précédentes, dans lequel ledit composé est administré audit patient pas plus tard que 7 j après ladite lésion musculosquelettique, notamment pas plus tard que 6 j après ladite lésion musculosquelettique.

8. Composé destiné à être utilisé dans un procédé de traitement d'une lésion musculosquelettique ou de prévention d'une cicatrisation retardée ou absence de cicatrisation d'une lésion musculosquelettique selon l'une quelconque des revendications précédentes, dans lequel ledit patient est compromis.

9. Composé destiné à être utilisé dans un procédé de traitement d'une lésion musculosquelettique ou de prévention d'une cicatrisation retardée ou absence de cicatrisation d'une lésion musculosquelettique selon l'une quelconque des revendications précédentes, dans lequel ladite lésion musculosquelettique est sélectionnée parmi une fracture osseuse, une lésion du tendon, une rupture du tendon, un muscle déchiré ou lésé.

10. Composition pharmaceutique destinée à être utilisée dans un procédé de traitement d'une lésion musculosquelettique ou de prévention d'une cicatrisation retardée ou absence de cicatrisation d'une lésion musculosquelettique comprenant un composé selon l'une quelconque des revendications précédentes.

11. Composition destinée à être utilisée dans un procédé de traitement d'une lésion musculosquelettique ou de prévention d'une cicatrisation retardée ou absence de cicatrisation d'une lésion musculosquelettique selon la revendication 10, dans laquelle ladite composition est formulée pour injection ou implantation.

12. Composition destinée à être utilisée dans un procédé de traitement d'une lésion musculosquelettique ou de prévention d'une cicatrisation retardée ou absence de cicatrisation d'une lésion musculosquelettique selon la revendication 10 ou 11, dans laquelle ladite composition comprend en outre un polymère biodégradable, notamment de la fibrine.

13. Composition destinée à être utilisée dans un procédé de traitement d'une lésion musculosquelettique ou de prévention d'une cicatrisation retardée ou absence de cicatrisation d'une lésion musculosquelettique selon la revendication 12, dans laquelle ladite composition est incorporée dans ledit polymère biodégradable et enrobée en option dudit polymère biodégradable ou d'un autre polymère biodégradable, ou essentiellement entourée d'une coque comprenant ou constituée dudit polymère biodégradable ou d'un autre polymère biodégradable.

14. Implant, notamment implant orthopédique, comprenant une composition selon l'une quelconque des revendications 10 à 13.
